# EUROPEAN PATENT APPLICATION

(11) **EP 4 775 953 A1**
(43) Date of publication of application: **15.07.2026**
(21) Application number: 26150011.0
(22) Date of filing: 02.01.2026
(51) Int. Cl.: G01N 21/03, G01N 21/39, G01J 3/42, G01J 3/02

(54) **MULTIBAND MULTI-PASS ABSORPTION SPECTROSCOPY GAS SENSING**

(30) Priority: 12.01.2025 US 202563744290 P; 12.12.2025 US 202519418518
(71) Applicant: Honeywell International Inc., Charlotte, NC 28202 (US)
(72) Inventor: FENG, Chen, Charlotte, 28202 (US); LEE, Seong Eyl, Charlotte, 28202 (US); SHAFAI, Moin S., Charlotte, 28202 (US)
(74) Representative: Haseltine Lake Kempner LLP

(57) **Abstract**

The present disclosure provides a multiband multi-pass absorption gas cell comprising a housing defining a chamber for receiving a gas sample to be analyzed. The cell comprises a first multiband mirror at a first end of the housing, defining inlet through-holes and comprising concentric dielectric coated zones corresponding to multiple wavelength bands. A second multiband mirror is positioned at the opposite end, comprising outlet through-holes and concentric dielectric coated zones corresponding to the wavelength bands. The cell includes inlet wedged windows positioned at the first end of the housing and outlet wedged windows positioned at the second end.

## Description

### CROSS-REFERENCE TO RELATED APPLICATIONS

This application claims priority to U.S. Provisional Patent Application No. 63/744,290, filed on January 12, 2025, which is incorporated herein by reference in its entirety.

### TECHNICAL FIELD

Embodiments of the present disclosure generally relate to gas sensing, and, more particularly, to compact multiband multi-pass absorption spectroscopy gas sensing.

### BACKGROUND

Applicant has identified many technical challenges associated with multi-pass absorption spectroscopy gas sensing. Through applied effort, ingenuity, and innovation, many of these identified problems have been solved by developing solutions that are included in embodiments of the present disclosure, many examples of which are described in detail herein.

### SUMMARY

According to an aspect of the present disclosure, a multiband multi-pass absorption gas cell is provided. The multiband multi-pass absorption gas cell comprises a housing defining a chamber for receiving a gas sample to be analyzed. The multiband multi-pass absorption gas cell comprises a first multiband mirror positioned at a first end of the housing. The first multiband mirror defines a plurality of inlet through-holes and comprises a first plurality of concentric dielectric coated zones corresponding to a plurality of wavelength bands. The multiband multi-pass absorption gas cell comprises a second multiband mirror positioned at a second end of the housing opposite the first end. The second multiband mirror comprises a plurality of outlet through-holes and a second plurality of concentric dielectric coated zones corresponding to the plurality of wavelength bands. The multiband multi-pass absorption gas cell comprises a plurality of inlet wedged windows positioned at the first end of the housing and a plurality of outlet wedged windows positioned at the second end of the housing.

According to other aspects of the present disclosure, the multiband multi-pass absorption gas cell may include one or more of the following features. The multiband multi-pass absorption gas cell may further comprise an inlet for introducing the gas sample into the chamber and an outlet for removing the gas sample from the chamber. Each of the first multiband mirror and the second multiband mirror may define an inner surface and an outer surface, wherein the inner surface of the first multiband mirror and the inner surface of the second multiband mirror face each other, and wherein the inner surface of the first multiband mirror may comprise the first plurality of concentric dielectric coated zones and the inner surface of the second multiband mirror may comprise the second plurality of concentric dielectric coated zones. The plurality of inlet wedged windows may be positioned adjacent the outer surface of the first multiband mirror and the plurality of outlet wedged windows may be positioned adjacent to the outer surface of the second multiband mirror. Each inlet through-hole of the plurality of inlet through-holes may be conical and may define a first opening on the inner surface of the first multiband mirror and a second opening on the outer surface of the first multiband mirror that is larger than the first opening. Each outlet through-hole of the plurality of outlet through-holes may be conical and may define a first opening on the inner surface of the second multiband mirror and a second opening on the outer surface of the second multiband mirror that is larger than the first opening. The plurality of inlet through-holes of the first multiband mirror may be defined within different dielectric coated zones of the first plurality of concentric dielectric coated zones, and the plurality of outlet through-holes of the second multiband mirror may be defined within different dielectric coated zones of the second plurality of concentric dielectric coated zones. The plurality of inlet through-holes of the first multiband mirror may be defined at different radial distances from a center of the first multiband mirror, and the plurality of outlet through-holes of the second multiband mirror may be defined at different radial distances from a center of the second multiband mirror. The first plurality of concentric dielectric coated zones and the second plurality of concentric dielectric coated zones may each comprise four concentric dielectric coated zones configured to provide greater than 99.5% reflectance in each of a plurality of wavelength bands. The plurality of wavelength bands may comprise ultraviolet wavelength band, visible wavelength band, near infrared wavelength band, and short infrared wavelength band. The first multiband mirror and the second multiband mirror may comprise a concave mirror.

According to another aspect of the present disclosure, a multiband multi-pass absorption gas sensing system is provided. The multiband multi-pass absorption gas sensing system comprises a broadband dual-comb laser source configured to emit light. The multiband multi-pass absorption gas sensing system comprises a fiber optic wavelength division multiplexer configured to generate a plurality of light beams from the light, wherein the plurality of light beams correspond to a plurality of wavelength bands. The multiband multi-pass absorption gas sensing system comprises a multiband multi-pass absorption gas cell configured to receive the plurality of light beams. The multiband multi-pass absorption gas cell comprises a housing defining a chamber for receiving a gas sample to be analyzed. The multiband multi-pass absorption gas cell comprises a first multiband mirror positioned at a first end of the housing. The first multiband mirror defines a plurality of inlet through-holes and comprises a first plurality of concentric dielectric coated zones corresponding to a plurality of wavelength bands. The multiband multi-pass absorption gas cell comprises a second multiband mirror positioned at a second end of the housing opposite the first end. The second multiband mirror comprises a plurality of outlet through-holes and a second plurality of concentric dielectric coated zones corresponding to the plurality of wavelength bands. The multiband multi-pass absorption gas cell comprises a plurality of inlet wedged windows positioned at the first end of the housing and a plurality of outlet wedged windows positioned at the second end of the housing.

According to other aspects of the present disclosure, the multiband multi-pass absorption gas sensing system may include one or more of the following features. The multiband multi-pass absorption gas sensing system may further comprise one or two fiber optic combiner coupler(s) configured to receive output light beams from the multiband multi-pass absorption gas cell and generate a single or two output beam(s). The multiband multi-pass absorption gas sensing system may further comprise one or two broadband photoelectric detector(s) and a dual comb spectroscopy processor. The multiband multi-pass absorption gas sensing system may further comprise an inlet for introducing the gas sample into the chamber and an outlet for removing the gas sample from the chamber. Each of the first multiband mirror and the second multiband mirror may define an inner surface and an outer surface, wherein the inner surface of the first multiband mirror and the inner surface of the second multiband mirror face each other, and wherein the inner surface of the first multiband mirror may comprise the first plurality of concentric dielectric coated zones and the inner surface of the second multiband mirror may comprise the second plurality of concentric dielectric coated zones. The plurality of inlet wedged windows may be positioned adjacent the outer surface of the first multiband mirror and the plurality of outlet wedged windows may be positioned adjacent to the outer surface of the second multiband mirror. Each inlet through-hole of the plurality of inlet through-holes may be conical and may define a first opening on the inner surface of the first multiband mirror and a second opening on the outer surface of the first multiband mirror that is larger than the first opening. Each outlet through-hole of the plurality of outlet through-holes may be conical and may define a first opening on the inner surface of the second multiband mirror and a second opening on the outer surface of the second multiband mirror that is larger than the first opening. The plurality of inlet through-holes of the first multiband mirror may be defined within different dielectric coated zones of the first plurality of concentric dielectric coated zones, and the plurality of outlet through-holes of the second multiband mirror may be defined within different dielectric coated zones of the second plurality of concentric dielectric coated zones.

### BRIEF DESCRIPTION OF FIGURES

Non-limiting and non-exhaustive examples are described with reference to the following figures.
FIG. 1 illustrates a perspective view of a multiband multi-pass absorption spectroscopy gas cell in accordance with at least some example embodiments of the present disclosure.
FIG. 2 illustrates a top-side view of the multiband multi-pass absorption spectroscopy gas cell of FIG. 1 in accordance with at least some example embodiments of the present disclosure.
FIGS. 3A-3B illustrate end views of a multiband mirror in accordance with at least some example embodiments of the present disclosure.
FIGS. 4A-4C illustrate various views of a multiband mirror in accordance with at least some example embodiments of the present disclosure.
FIGS. 5A-C illustrate various views of a wedged window in accordance with at least some example embodiments of the present disclosure.
FIG. 5D illustrates a front view of a multiband mirror showing concentric dielectric coated zones in accordance with at least some example embodiments of the present disclosure.
FIG. 6 illustrates a system diagram of a multiband multi-pass absorption spectroscopy gas sensing system in accordance with at least some example embodiments of the present disclosure.
FIGS. 7A-B illustrate reflection patterns in accordance with at least some embodiments of the present disclosure.
FIG. 8 illustrates reflection patterns in accordance with at least some example embodiments of the present disclosure.
FIG. 9A depicts a dielectric coated mirror reflectance plot in accordance with at least some example embodiments of the present disclosure.
FIG. 9B depicts a mirror reflectivity plot in accordance with at least some example embodiments of the present disclosure.
FIG. 9C depicts gold coating reflectance plot.
FIG. 10 illustrates a block diagram of a multiband multi-pass absorption spectroscopy gas sensing system in accordance with at least some example embodiments of the present disclosure.

### DETAILED DESCRIPTION

Some embodiments of the present disclosure will now be described more fully hereinafter with reference to the accompanying figures, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

The term "or" is used herein in both the alternative and conjunctive sense, unless otherwise indicated. The terms "illustrative" and "example" are used to be examples with no indication of quality level. Terms such as "computing," "determining," "generating," and/or similar words are used herein interchangeably to refer to the creation, modification, or identification of data. Further, "based on," "based on in part on," "based at least on," "based upon," and/or similar words are used herein interchangeably in an open-ended manner such that they do not indicate being based only on or based solely on the referenced element or elements unless so indicated.

As used herein, terms such as "front," "rear," "top," "bottom," "left," "right," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "example" or "exemplary" is not necessarily to be construed as preferred or advantageous over other implementations.

The term "electrically connected," "electronically coupled," "electronically coupling," "electronically couple," "in communication with," "in electronic communication with," or "connected" in the present disclosure refers to two or more elements or components being connected through wired means and/or wireless means, such that signals, electrical voltage/current, data and/or information may be transmitted to and/or received from these elements or components.

The term "component" may refer to an article, a device, or an apparatus that may comprise one or more surfaces, portions, layers and/or elements. For example, an example component may comprise one or more substrates that may provide underlying layer(s) for the component and may comprise one or more elements that may form part of and/or are disposed on top of the substrate. the term "element" may refer to an article, a device, or an apparatus that may provide one or more functionalities.

### Overview

Multi-pass absorption spectroscopy gas sensing technology utilizes multiple reflections of light beams between mirrors in a gas cell to multiply the gas absorption path length, thereby enhancing the sensitivity of gas detection and analysis. Light beams are reflected back and forth between mirrors positioned at opposite ends of a chamber comprising the gas sample to be analyzed. The extended optical path length achieved through multiple reflections allows for improved detection of low concentration gases and gases with weak absorption features.

Conventional multi-pass absorption gas cells face several technical challenges that limit their performance and applicability. A primary challenge involves mirror reflection losses that occur with each reflection of the light beam. Conventional gold-coated mirrors may provide no more than 95% reflectance in short infrared wavelengths and approximately 90% reflectance in near infrared wavelengths. The reflectance performance of gold-coated mirrors deteriorates further at shorter wavelengths, with reflectance values dropping below 90% in most visible wavelengths and becoming unusable in ultraviolet wavelengths. These low reflectance values limit the number of reflections that may be achieved before the signal becomes too weak for accurate detection, thereby constraining the enhancement factor and sensitivity of the gas sensing system.

The reflectance limitations of conventional mirrors create additional challenges for broadband gas sensing applications. Different gas species exhibit absorption features at different wavelengths across the electromagnetic spectrum, from ultraviolet to infrared regions. To achieve comprehensive gas detection capabilities, a gas sensing system may need to operate across multiple wavelength bands simultaneously. However, conventional single-coating mirror systems cannot provide adequate reflectance across such a broad spectral range, limiting their effectiveness for multiband applications.

Various embodiments of the present disclosure provide a multiband multi-pass absorption spectroscopy gas cell that addresses the above technical challenges through the implementation of multiband dielectric mirrors with concentric dielectric coated zones that provide greater than 99.5% reflectance across multiple wavelength bands simultaneously. In various embodiments, each dielectric coated zone may be optimized for a specific wavelength band.

In various embodiments, the multiband multi-pass absorption spectroscopy gas cell includes a first multiband mirror and a second multiband mirror positioned opposite each other to define a chamber for receiving the gas sample. In various embodiments, each multiband mirror has multiple concentric dielectric coated zones, with each zone providing high reflectance in a specific wavelength band. In various embodiments, the gas cell includes inlet and outlet through-holes in the mirrors to allow light beams to enter and exit the chamber, along with corresponding wedged windows to minimize back reflection interference. The multiband approach allows a single gas cell to accommodate multiple wavelength bands, from ultraviolet to infrared wavelengths, without the reflectance limitations associated with conventional mirror coatings.

Various embodiments of the present disclosure provide a multiband multi-pass absorption spectroscopy gas sensing system incorporating a multiband multi-absorption gas spectroscopy gas cell, as described herein, along with one or more optical components. In various embodiments, the system includes a broadband light source, wavelength division multiplexing components to separate the broadband light into multiple wavelength bands, and detection components to analyze the output beams after they have traversed the gas cell. In various embodiments, the system includes processing components to analyze the spectroscopic data and to determine the presence and concentration of target gases. Various embodiments of the present disclosure provide a method multiband multi-pass absorption spectroscopy gas sensing that involves dividing broadband light into multiple wavelength bands, introducing these bands into the gas cell through the multiband mirrors, allowing multiple reflections to occur within the chamber, and subsequently detecting and analyzing the output beams to determine gas absorption characteristics. This method may enable simultaneous analysis of multiple gas species across different wavelength bands using a single compact gas cell system.

### EXAMPLE SYSTEMS, APPARATUSES, AND METHODS OF THE DISCLOSURE

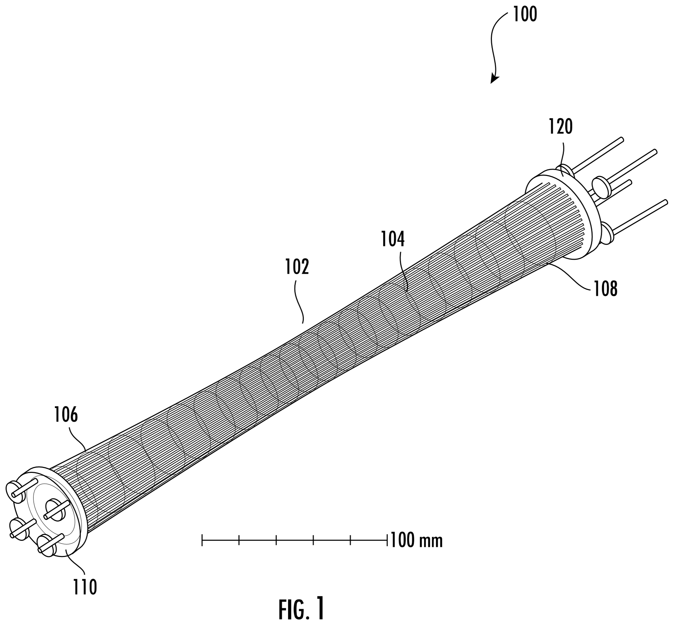

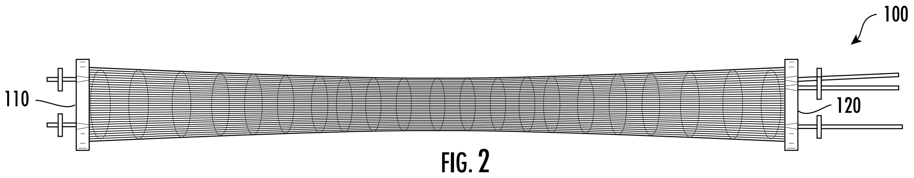

Referring to FIGS. 1 and 2, a multiband multi-pass absorption spectroscopy gas cell 100 (gas cell 100) may be configured to enable enhanced gas detection through multiple light beam reflections. The multiband multi-pass absorption spectroscopy gas cell 100 may comprise a cell housing 102 defining an interior. In some embodiments, the cell housing 102 may define an interior with a substantially cylindrical shape. In some embodiments, the cell housing may include a cylindrical body or substantially cylindrical body that provides structural support for components of the gas cell 100. In some embodiments, the gas cell 100 further comprises a pressure-temperature sensor (which may be disposed on the cell housing). The pressure-temperature sensor may be configured to measure, detect, collect, and/or the like the gas flow pressure and temperature reading for gas sensing data analysis.

As shown in FIGS. 1 and 2, the multiband multi-pass absorption spectroscopy gas cell 100 may include a first multiband mirror 110 positioned at one end of the cylindrical structure. A second multiband mirror 120 may be positioned at an opposite end of the cylindrical structure. The first multiband mirror 110 and the second multiband mirror 120 may be substantially identical in design and construction. The first multiband mirror 110 and the second multiband mirror 120 may be affixed or coupled to opposing ends of the cell housing.

The first multiband mirror 110 and the second multiband mirror 120 may be positioned opposite each other to define a chamber 104 therebetween. The chamber 104 may be configured for receiving gas (e.g., gas samples) to be analyzed. In various embodiments, the first multiband mirror 110 and the second multiband mirror 120 may be have a substantially circular shape. The first multiband mirror 110 and the second multiband mirror 120 may be concave mirrors that face each other across the chamber 104. For example, the first multiband mirror 110 and the second multiband mirror 120 may each define an inner surface and an outer surface, wherein the inner surface of the first multiband mirror and the inner surface of the second multiband mirror face each other. The concave configuration of the first multiband mirror 110 and the second multiband mirror 120 may enable controlled reflection patterns for the light beams traversing the chamber 104.

The multiband multi-pass absorption spectroscopy gas cell 100 may include a gas inlet 106 (e.g., gas inlet portion) via which gas to be analyzed is added to the chamber 104. The multiband multi-pass absorption spectroscopy gas cell 100 may also include a gas outlet 108 (e.g., gas outlet portion) via which the gas sample is removed from the chamber 104. The gas inlet and gas outlet portions may be configured to allow gas to flow through the chamber 104 for detection and analysis.

The configuration of the first multiband mirror 110 and the second multiband mirror 120 may enable multi-pass absorption spectroscopy by reflecting light beams multiple times between the mirrors. The multiple reflections may increase the optical path length for enhanced gas detection sensitivity. As light beams traverse the chamber 104 and undergo multiple reflections, the extended optical path length may allow for improved detection of gases with low concentrations or weak absorption features.

As shown in FIGS 1 and 2, the multiband multi-pass absorption spectroscopy gas cell 100 may have an elongated structure along its longitudinal axis. The first multiband mirror 110 and the second multiband mirror 120 may be spaced apart along the length of the gas cell to define an elongated chamber (e.g., chamber 104) between the mirrors. The first multiband mirror 110 and the second multiband mirror 120 may be oriented to face each other across the length of the chamber 104 or housing 102. The spacing between the first multiband mirror 110 and the second multiband mirror 120 may provide sufficient distance for multiple light beam reflections to occur within the chamber. The orientation of the mirrors may enable light beams to travel back and forth between the mirror surfaces multiple times before exiting the gas cell.

As illustrated in FIG. 2, light may enter the multiband multi-pass absorption spectroscopy gas cell 100 through the first multiband mirror 110. The light beams may then travel within the chamber 104 and undergo multiple reflections between the first multiband mirror 110 and the second multiband mirror 120. The multiple reflections may occur in a controlled pattern that maximizes the optical path length while avoiding interference between different light beams.

The light beams may exit the multiband multi-pass absorption spectroscopy gas cell 100 through the second multiband mirror 120 after completing the multiple reflection process. The extended optical path length achieved through the multiple reflections between the first multiband mirror 110 and the second multiband mirror 120 may provide enhanced sensitivity for gas absorption spectroscopy measurements. The longitudinal configuration may enable the gas cell to achieve a high number of reflections within a compact form factor, making the system suitable for various gas sensing applications.

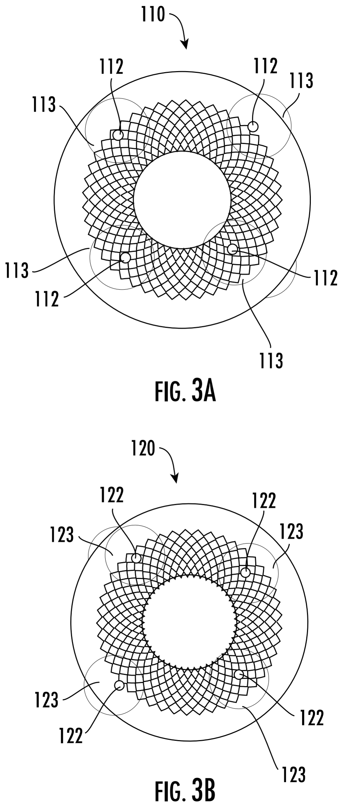

Referring to FIG. 3A and FIG. 3B, end views of multiband mirrors in accordance with at least some embodiments of the present disclosure is provided. Specifically, FIG. 3A shows an end view of the first multiband mirror 110 and FIG. 3B shows an end view of the second multiband mirror 120. The multiband mirror 110 and multiband mirror 120 define inlet and outlet portions, respectively, that enable multiband spectroscopy operations as described herein.

As shown in FIG. 3A, the first multiband mirror 110 may define a plurality of inlet through-holes 112 that extend through the mirror body. The inlet through-holes 112 may be arranged in a pattern on the mirror surface to accommodate different wavelength bands. For example, each of the inlet through-holes 112 may be positioned or otherwise defined at a different radial distance from the center of the first multiband mirror 110, with the positioning corresponding to different concentric coated dielectric zones on the mirror surface (e.g., inner surface), further described below. The arrangement of the inlet through-holes 112 may enable multiple light beams to be introduced into the chamber 104 at different spectral bands.

The first multiband mirror 110 may include a plurality of inlet windows 113 that correspond to the plurality of inlet through-holes 112. For example, the gas cell 100 may include a plurality of inlet windows 113, wherein each inlet window 113 is positioned adjacent to a respective inlet through-hole 112 to allow light beams to enter the chamber 104. Each inlet window 113 may be positioned on the outer surface of the first multiband mirror 110 adjacent to the respective inlet through-hole 112. The inlet windows 113 may be configured to transmit light beams while maintaining the structural integrity of the chamber 104. For example, the inlet windows may comprise optical transmission elements configured to allow light beams of different wavelength bands to enter the chamber while maintaining the structural integrity and optical performance of the gas cell 100. The inlet window may be constructed of an optically transparent material such as fused silica or other similar near-infrared optical material that provides suitable optical transmission characteristics across the relevant wavelength bands. In various embodiments, the inlet window may have a wedged geometry with an angled surface configured to minimize back reflection interference when light passes through the window into the chamber.

With continued reference to FIG. 3A, the positioning of the inlet through-holes 112 and inlet windows 113 at different radial distances may correspond to the concentric dielectric coated zones on the first multiband mirror 110. Each dielectric coated zone may be optimized for a specific wavelength band, and the corresponding inlet through-holes 112 and inlet windows 113 may be positioned to align with corresponding zones. The inlet through-holes 112 may be defined within the different dielectric coated zones on the first multiband mirror 110. The radial arrangement may enable multiple wavelength bands to enter the chamber 104 without interference between different spectral channels.

As illustrated in FIG. 3B, the second multiband mirror 120 may define a plurality of outlet through-holes 122 that extend through the mirror body. The outlet through-holes 122 may be arranged in a pattern that corresponds to the arrangement of the inlet through-holes 112 on the first multiband mirror 110. Each of the outlet through-holes 122 may be positioned or otherwise defined at a different radial distance from the center of the second multiband mirror 120, with the positioning corresponding to different concentric dielectric coated zones on the mirror surface (e.g., inner surface).

The second multiband mirror 120 may include a plurality of outlet windows 123 that correspond to the plurality of outlet through-holes 122. For example, the gas cell 100 may include a plurality of outlet windows 123, wherein each outlet window 123 is positioned adjacent to a respective outlet through-hole 122 to allow light beams to exit the chamber 104 after multiple reflections between the first multiband mirror 110 and the second multiband mirror 120. The outlet windows 123 may be configured to transmit the light beam exiting the gas cell 100 while maintaining the optical performance of the gas cell. The inlet and outlet windows may be positioned adjacent the outer surface of the respective multiband mirrors.

The arrangement of the outlet through-holes 122 and outlet windows 123 at different radial distances may correspond to the concentric dielectric coated zones on the second multiband mirror 120. The outlet through-holes 122 may be defined within the different dielectric coated zones on the multiband mirror 120. The radial positioning may enable multiple wavelength bands to exit the chamber 104 after completing the multi-pass reflection process. The outlet configuration may maintain the spectral separation of different wavelength bands, allowing for subsequent detection and analysis of each band. The radial arrangement may enable different wavelength bands to access their respective coating zones without interference.

In some embodiments, the inlet through-holes 112 and the outlet through-holes 122 may each be conical with larger openings on exterior sides (e.g., outer surface) of their respective mirrors. In this regard, each inlet through-hole may be conical and define a first opening on the inner surface of the first multiband mirror and a second opening on the outer surface of the first multiband mirror that is larger than the first opening. Each outlet through-hole may be conical and define a first opening on the inner surface of the second multiband mirror and a second opening on the outer surface of the second multiband mirror that is larger than the first opening. The conical configuration may facilitate the introduction and extraction of light beams while minimizing optical losses. The larger openings on the exterior sides (e.g., outer surface) may provide improved coupling with external optical components, while the smaller openings on the interior sides (e.g., inner surface) may maintain the optical characteristics within the chamber 104.

The plurality of inlet windows 113 and plurality of outlet windows 123 may have a wedged geometry. In this this regard, the multiband multi-pass absorption spectroscopy gas cell 100 may include a first set of wedged windows (e.g., plurality of inlet wedged windows 113) corresponding to the plurality of inlet through-holes 112 and a second set of wedged windows (e.g., plurality of outlet wedged windows 123) corresponding to the plurality of outlet through-holes 122. The plurality of inlet wedged windows 113 may be configured to minimize back reflection interference when light beams enter the chamber 104. The plurality of outlet wedged windows 123 may be configured to minimize back reflection interference when light beams exit the chamber 104.

The wedged windows may comprise a wedge surface that is angled relative to an opposing surface of the window, creating a non-parallel configuration that reduces back reflection interference. The angular orientation of the wedge surface may cause any reflected light to be directed away from the optical path, thereby preventing interference with incident light beams or the multi-pass reflection process within the chamber. The wedged windows may be constructed of optically transparent materials such as fused silica or other similar near-infrared optical materials that provide suitable optical transmission characteristics across relevant wavelength bands while maintaining structural integrity and optical performance of the gas cell.

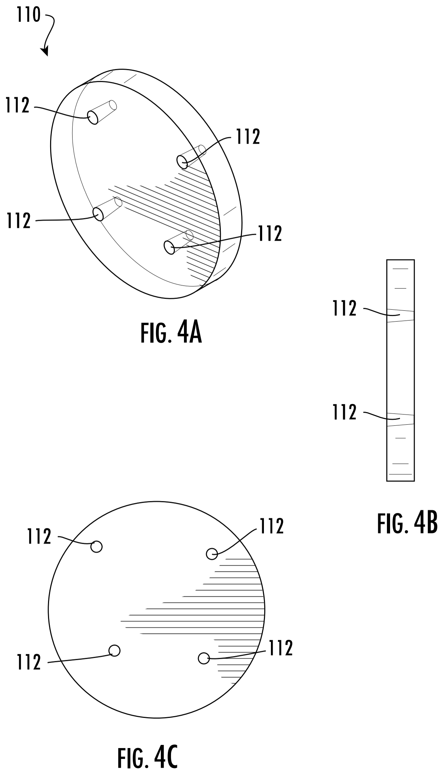

Referring to FIGS. 4A-4C, different views of a multiband mirror (multiband mirror 110 or multiband mirror 120) in accordance with at least some embodiments of the present disclosure is provided. Specifically, FIGS. 4A-4C illustrate the structural configuration and optical characteristics that enable multiband spectroscopy operations. The multiband mirror shown in FIGS. 4A-4C may represent the first multiband mirror 110 or the second multiband mirror 120 which may have substantially similar construction and features.

As shown in FIG. 4A, a multiband mirror, as described herein, may have a concave reflective surface that curves inward to provide controlled reflection characteristics for light beams within the gas cell 100. The concave configuration may enable the multiband mirror to focus and direct light beams in predetermined patterns during the multi-pass reflection process. The concave reflective surface may be shaped to optimize the reflection geometry for achieving multiple reflections without interference between different light paths.

As described above, the multiband mirror may define a plurality of inlet through-holes 112 that extend through the mirror body from the exterior surface (outer surface) to the concave reflective surface (e.g., concave reflective inner surface). As illustrated in FIG. 4A, the inlet through-holes 112 may be distributed across the mirror surface in a pattern that corresponds to the concentric dielectric coated zones. Each inlet through-hole 112 may provide a passage for light beams to enter the chamber of the gas cell. The positioning of the inlet through-holes 112 may be coordinated with the concentric dielectric coated zones to ensure that each wavelength band enters the gas cell at the appropriate location on the mirror surface.

The multiband mirror may have a concave curvature that extends across the multiband mirror surface to provide the desired reflection characteristics. The concave reflective surface may be configured to support multiple concentric dielectric coatings that enable multiband spectroscopy applications. Each concentric dielectric coated zone may be optimized for a specific wavelength range, allowing the mirror to achieve high reflectance values across different spectral bands simultaneously. The concave geometry may provide a suitable substrate for applying the dielectric coatings while maintaining the optical performance characteristics needed for multi-pass absorption spectroscopy.

The multiband mirrors may be constructed of fused silica or other similar near-infrared optical material that provides suitable optical and mechanical properties for the gas cell application. Fused silica may offer low thermal expansion characteristics, high optical transmission, and compatibility with dielectric coating processes. The material properties of fused silica may enable the mirror to maintain dimensional stability and optical performance across a range of operating conditions.

The concave reflective surface may be precisely shaped to achieve the desired reflection patterns for multi-pass absorption spectroscopy. The surface curvature may be designed to ensure that light beams undergo multiple reflections in a controlled manner that maximizes the optical path length while avoiding interference between different reflection paths. The concave geometry may also facilitate the application of multiple concentric dielectric coatings, with each coating zone providing optimized reflectance for its corresponding wavelength band.

The inlet through-holes may be formed through precision machining or other suitable manufacturing processes that maintain the optical quality of the mirror surface. The conical geometry of each inlet through-hole may be carefully controlled to ensure proper optical coupling and minimal losses during light transmission. The transition from the larger exterior opening to the smaller interior opening may be smooth to avoid optical aberrations or scattering that could degrade the performance of the gas cell.

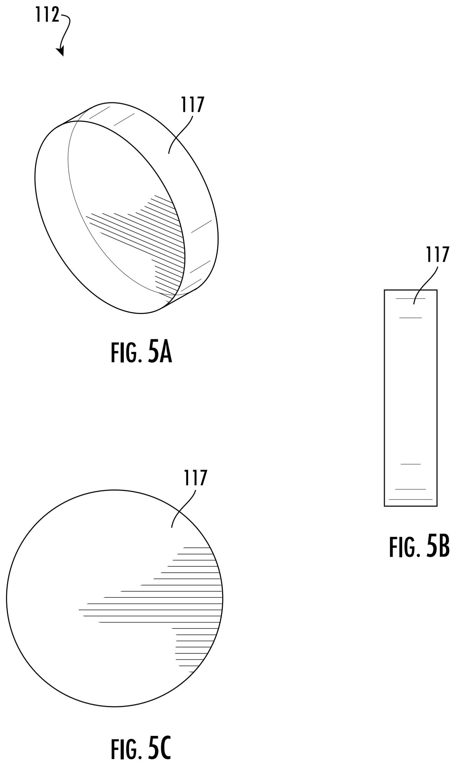

Referring to Figs. 5A-5C, the wedged window configuration may provide optical characteristics that minimize interference and enhance the performance of the multiband multi-pass absorption spectroscopy gas cell 100. The wedged windows (e.g, inlet wedged window 113 and outlet wedged window 123) may be positioned at the inlet through-holes 112 and outlet through-holes 122 to facilitate the transmission of light beams while reducing unwanted optical effects that could degrade the spectroscopic measurements.

As shown in FIG. 5A, a wedged window may have a generally cylindrical or disk-like shape with a circular cross-section that provides compatibility with the inlet through-holes and outlet through-holes of the multiband mirrors. The cylindrical configuration may facilitate mounting and alignment of the wedged window within the gas cell assembly. The wedged window may include a wedge surface 117 that is angled relative to an opposing surface of the window. The wedge surface 117 may be oriented at a specific angle to reduce or eliminate back reflection interference when light passes through the window. The angled configuration of the wedge surface 117 may cause any reflected light to be directed away from the optical path, thereby preventing interference with the incident light beams or the multi-pass reflection process within the chamber 104.

The wedged windows may be configured to be positioned at one of the inlet through-holes 112 or outlet through-holes 122 of the first multiband mirror 110 or second multiband mirror 120, as described above. The positioning of the wedged window may allow light beams to enter or exit the chamber 104 while minimizing optical interference from reflected light. The wedge surface 117 may be oriented to direct any back reflections away from the optical paths within the gas cell. The wedged windows may have an angular relationship between the wedge surface 117 and the opposing surface of the wedged window. The wedge surface 117 may be angled to create a non-parallel configuration that reduces back reflection interference. The angular orientation may be selected to optimize the transmission characteristics while minimizing unwanted reflections that could interfere with the spectroscopic measurements. The wedged windows may have circular aperture that allows light transmission. The reduction of back reflection interference may improve the signal-to-noise ratio of the spectroscopic measurements and enhance the overall performance of the multiband multi-pass absorption spectroscopy gas cell 100. The circular configuration may provide uniform optical characteristics across the aperture and facilitate alignment with the inlet through-holes 112 or outlet through-holes 122.

The wedge surface 117 may be formed through precision optical polishing or other suitable manufacturing processes that maintain the required surface quality and angular accuracy. The surface finish of the wedge surface 117 may be controlled to minimize scattering and maintain high optical transmission. The angular tolerance of the wedge surface 117 may be precisely controlled to ensure consistent performance across all wedged windows in the gas cell assembly.

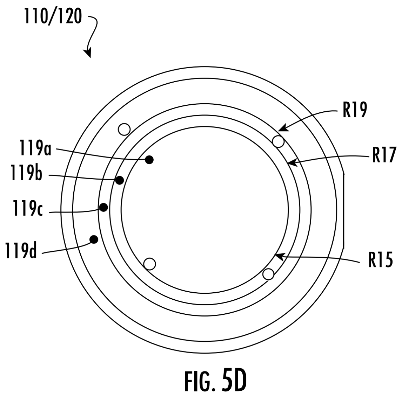

Referring to FIG. 5D, an example multiband mirror (e.g., multiband mirror 110 or multiband mirror 120) illustrating the concentric dielectric coated zone configuration thereof in accordance with at least some embodiments of the present disclosure is provided. FIG. 5D illustrates a circular cross-sectional view of the multiband mirror depicting a plurality of concentric dielectric coated zones with different radii that define the boundaries for different dielectric coating types. Each concentric coated zone may represent a band of the multiband mirrors. For example, each concentric coated zone may be referred to as a concentric coated band. The concentric arrangement may enable the multiband multi-pass absorption spectroscopy gas cell 100 to accommodate multiple spectral bands simultaneously while maintaining high reflectance characteristics across a broad wavelength range.

In some embodiments, the multiband mirrors may each comprise four concentric dielectric coated zones. For example, in some embodiments, the first multiband mirror 110 are the second multiband mirror 120 may be coated with high reflective dielectric coating in four concentric zones to cover four spectral bands with a high reflectance such as, for example, 99.5% reflectance. In some embodiments, the first multiband mirror 110 and the second multiband mirror 120 may be coated in more or less than four concentric zones.

In some embodiments, the concentric dielectric coated zones may be optimized for specific wavelength bands. For example, In some embodiments, the dielectric coated zones in the respective multiband mirrors may have different dielectric coating configurations, different coating types, or the like that optimizes the concentric dielectric coated bands for specific wavelength bands.

In some embodiments, the multiband mirrors may include a first concentric dielectric coated zone 119a (e.g., first concentric dielectric coated band) positioned closest to the center of the circular cross-section of the multiband mirror relative to other dielectric coated zones. In some embodiments, the first concentric dielectric coated zone 119a may be positioned at the center of the circular cross-section of the multiband mirror. In some embodiments, the first concentric dielectric coated zone 119a may occupy the central region of the multiband mirror surface and may be configured to provide high reflectance for a specific wavelength band. In some embodiments, the first concentric dielectric coated zone 119a may be optimized for ultraviolet wavelengths, providing reflectance characteristics that enable effective multi-pass absorption spectroscopy in the ultraviolet spectral range. For example, the first concentric dielectric coated zone 119a may be configured to provide high reflectance for near ultraviolet and blue wavelengths, such as for example, in the range of 0.35 to 0.45 microns, for multiband multi-pass absorption spectroscopy gas sensing applications.

The multiband mirrors may include a second dielectric concentric coated zone 119b (e.g., second concentric dielectric coated band) that surrounds the first concentric dielectric coated zone 119a in a concentric arrangement. The second dielectric concentric coated zone 119b may be positioned radially outward from the first concentric dielectric coated zone 119a. In some embodiments, the second dielectric concentric coated zone 119b may be configured to provide high reflectance for a different wavelength band than the first concentric dielectric coated zone 119a. For example, in some embodiments, the second dielectric concentric coated zone 119b may be optimized for visible wavelengths, enabling effective spectroscopy operations in the visible spectral range. The second dielectric concentric coated zone 119b may be configured to provide high reflectance for near visible wavelengths, such as for example, in the range of 0.4 to 0.9 microns for multiband multi-pass absorption spectroscopy gas sensing applications.

The multiband mirrors may include a third concentric dielectric coated zone 119c (e.g., third concentric dielectric coated band) that surrounds the second dielectric concentric coated zone 119b in the concentric arrangement. The third concentric dielectric coated zone 119c may be positioned radially outward from the second dielectric concentric coated zone 119b. In some embodiments, the third concentric dielectric coated zone 119c may be configured to provide high reflectance for near infrared wavelengths. The third concentric dielectric coated zone 119c may enable the multiband multi-pass absorption spectroscopy gas cell 100 to perform spectroscopy operations in the near infrared spectral range with high efficiency. For example, the third concentric dielectric coated zone 119c may be configured to provide high reflectance for near infrared wavelengths, such as for example in the range of 0.8 to 1.25um, for multiband multi-pass absorption spectroscopy gas sensing applications.

The multiband mirror may include a fourth concentric dielectric coated zone 119d (e.g., fourth concentric dielectric coated band) that surrounds the third concentric dielectric coated zone 119c as the outermost zone in the concentric arrangement. The fourth concentric dielectric coated zone 119d may be positioned at the outermost radial location relative to the other coating zones. In some embodiments, the fourth concentric dielectric coated band may be configured to provide high reflectance for short infrared wavelengths. For example, the fourth concentric dielectric coated zone 119d may be configured to provide high reflectance for short infrared wavelengths, such as for example, in the range of 1.25 to 1.75 microns, for multiband multi-pass absorption spectroscopy gas sensing applications. The fourth concentric dielectric coated zone 119d may complete the multiband configuration by enabling spectroscopy operations in the short infrared spectral range.

The concentric dielectric coated zones (e.g., high reflective concentric dielectric coated bands) may be defined by specific radii that establish the boundaries between different coating zones. As shown in FIG. 5D, a first radius R15 may define the boundary between the first concentric dielectric coated zone 119a and the second dielectric concentric coated zone 119b, establishing the outer limit of the central coating region. The first radius R15 may determine the area of the mirror surface that is optimized for the first wavelength band, such as ultraviolet wavelengths. The first radius R15 may establish the transition point between the coating zone optimized for near ultraviolet and blue wavelengths and the coating zone optimized for visible wavelengths. The positioning of the first radius R15 may be selected to provide appropriate surface area for each coating zone based on the optical requirements of the respective wavelength bands.

A second radius R17 may define the boundary between the second dielectric concentric coated zone 119b and the third concentric dielectric coated zone 119c. The second radius R17 may establish the transition point between the coating zone optimized for visible wavelengths and the coating zone optimized for near infrared wavelengths. The positioning of the second radius R17 may be selected to provide appropriate surface area for each coating zone based on the optical requirements of the respective wavelength bands.

A third radius R19 may define the boundary between the third concentric dielectric coated zone 119c and the fourth concentric dielectric coated zone 119d. The third radius R19 may establish the transition point between the coating zone optimized for near infrared wavelengths and the coating zone optimized for short infrared wavelengths. The positioning of the third radius R19 may be selected to provide appropriate surface area for each coating zone based on the optical requirements of the respective wavelength bands.

The arrangement of the concentric dielectric coated zones may enable each coating zone to correspond to a different wavelength band, allowing the multiband multi-pass absorption spectroscopy gas cell 100 to achieve high reflectance across a broad spectrum from ultraviolet to infrared wavelengths. The four concentric dielectric coated zones may cover four spectral bands (e.g., four wavelength bands) with greater than 99.5% reflectance, providing enhanced performance compared to conventional mirror systems. The four spectral bands may comprise ultraviolet, visible, near infrared, and short infrared wavelengths, enabling comprehensive gas detection capabilities across multiple spectral regions.

For example, the first concentric dielectric coated zone 119a may represent a first dielectric coating band corresponding to a first wavelength band (e.g., ultraviolet band), the second dielectric concentric coated zone 119b may represent a second dielectric coating band corresponding to a second wavelength band (e.g., visible wavelength band), the third concentric dielectric coated zone 119c may represent a third dielectric coating band corresponding to a third wavelength band (e.g., near infrared wavelength band), the fourth concentric dielectric coated zone 119d may represent a fourth dielectric coating band corresponding to a fourth wavelength band (e.g., short infrared wavelength band).

In some embodiments, at least one of the coating types applied to the concentric zones may be a narrowband, ultra high reflectivity dielectric coating selected such that a resonant light cavity may be formed between the respective coatings of the first multiband mirror 110 and the respective coatings of the second multiband mirror 120. The narrowband dielectric coating may provide high reflectance values within a specific wavelength range, enabling enhanced sensitivity for gas detection applications that target specific absorption lines.

In some embodiments, at least one of the coating types applied to the concentric zones may have a reflectance of at least 99.99%, providing near-ideal reflection characteristics that minimize losses during the multi-pass reflection process. The ultra-high reflectance values may enable a greater number of reflections to occur before the signal strength becomes insufficient for accurate detection, thereby extending the effective optical path length and enhancing the sensitivity of the gas sensing system.

In some embodiments, at least one of the coating types applied to the concentric zones may be a broadband, metal coating selected such that a multi-pass light cavity may be formed between the respective coatings of the first multiband mirror 110 and the respective coatings of the second multiband mirror 120. The broadband metal coating may provide adequate reflectance across a wider wavelength range, enabling multi-pass operations for applications that require broader spectral coverage within a single coating zone.

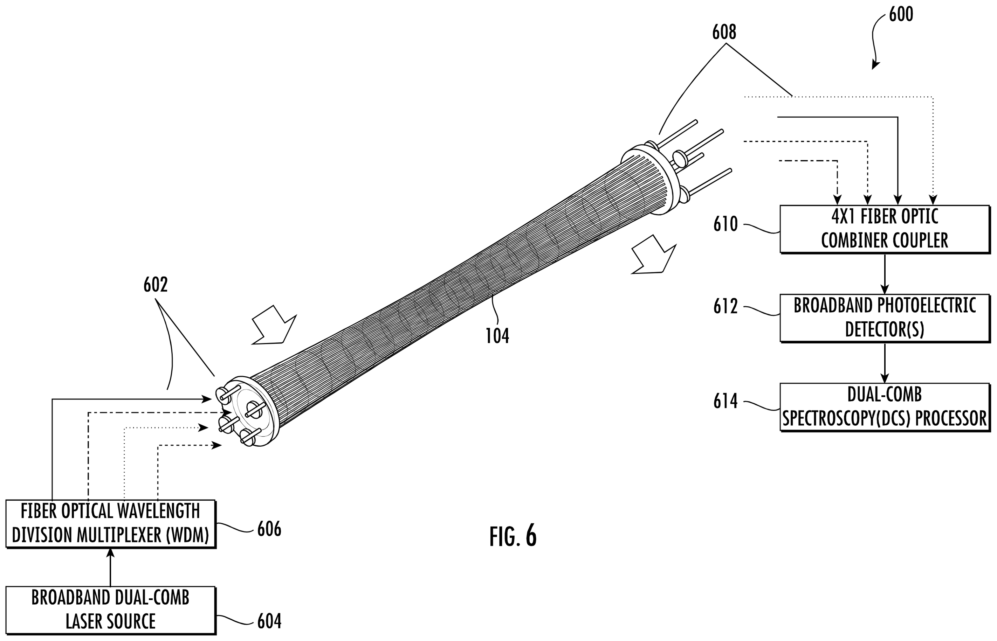

Referring to FIG. 6, a multiband multi-pass absorption spectroscopy gas sensing system 600 in accordance with at least some embodiments of the present disclosure is provide. As shown in FIG. 6, the multiband multi-pass absorption spectroscopy gas sensing system 600 may include multiband multi-pass absorption spectroscopy gas cell, such as the multiband multi-pass absorption spectroscopy gas cell 100 described above, and one or more optical components to provide comprehensive gas detection and analysis across multiple wavelength bands. The system 600 may provide enhanced sensitivity and broad spectral coverage through the integration of advanced optical components with the multiband gas cell configuration. The system 600 may be configured to enable simultaneous analysis of multiple gas species while maintaining high detection sensitivity through extended optical path lengths. The concentric dielectric coated zone configuration of the multiband mirrors of the gas cell 100, as described above, may enable the multiband multi-pass absorption spectroscopy gas sensing system to utilize four concentric dielectric coated zones that cover four spectral bands with greater than 99.5% reflectance. The system configuration may incorporate the multiband mirror design to achieve enhanced performance across ultraviolet, visible, near infrared, and short infrared wavelengths simultaneously within a single gas cell assembly.

The multiband multi-pass absorption spectroscopy gas sensing system 600 may include a broadband dual-comb laser source 604 that generates light for spectroscopy analysis. The broadband dual-comb laser source 604 may emit coherent light across a broad wavelength range that encompasses multiple spectral bands to support comprehensive gas detection. The broadband dual-comb laser source 604 may provide stable, high-quality optical output that enables precise spectroscopic measurements across ultraviolet, visible, near infrared, and short infrared wavelengths.

As shown in FIG. 6, the system 600 may include a fiber optic wavelength division multiplexer 606 that may be configured to divide light from the broadband dual-comb laser source 604 into a plurality of input beams. The fiber optic wavelength division multiplexer 606 may receive the broadband light output from the broadband dual-comb laser source 604 and separate the light into distinct wavelength bands (e.g., different wavelength band input beams) that correspond to the concentric dielectric coated zones of the multiband mirrors. The fiber optic wavelength division multiplexer 606 may divide the light into the plurality of input beams 602 without amplitude drop in each beam, ensuring that each beam/wavelength band maintains sufficient optical power for effective spectroscopic analysis.

In this regard, the fiber optic wavelength division multiplexer 606 may output input beams 602 that are directed into the multiband multi-pass absorption spectroscopy gas cell 100, wherein the input beams 602 comprise multiple wavelength bands that have been separated from the original broadband light source. Each of the input beams 602 may correspond to a specific spectral band that will interact with a corresponding concentric dielectric coated zone on the first multiband mirror 110 and the second multiband mirror 120. The input beams 602 may be fed or otherwise input into the chamber 104 of the multiband multi-pass absorption spectroscopy gas cell 100 through the inlet wedged windows 113 and the inlet through-holes 112.

With continued reference to FIG. 6, each input beam of the input beams 602 may pass through a respective inlet window 113 of the plurality of inlet wedged windows and through a respective inlet through-hole of the inlet through-holes 112. The input beams 602 may travel within the chamber 104 toward the second multiband mirror 120 without back reflection interference due to the wedged window configuration. The input beams 602 may enter the chamber 104 at different radial positions that correspond to the concentric dielectric coated zones, enabling each wavelength band to interact with its optimized coating zone.

The input beams 602 may undergo multiple reflections within the chamber 104 to enhance the optical path length for improved gas detection sensitivity. Due to the high reflectance of the inner surfaces of the first multiband mirror 110 and the second multiband mirror 120, the input beams 602 may reflect back and forth between the mirrors according to a pattern that avoids collisions and interference between and among the reflections. The inner surfaces of the first multiband mirror 110 and the second multiband mirror 120 may be shaped and positioned such that the input beams 602 are reflected back and forth in a controlled manner that maximizes the optical path length while maintaining spectral separation.

In some embodiments, each input beam of the input beams 602 may be reflected approximately eighty times between the first multiband mirror 110 and the second multiband mirror 120 to enhance the absorption gas sensing sensitivity. The multiple reflections may extend the effective optical path length compared to a single-pass configuration, enabling detection of gases with low concentrations or weak absorption features. The eighty reflections may provide an enhancement factor that improves the signal-to-noise ratio and detection limits of the system 600.

As illustrated in FIG. 6, some amount of the input beams 602 may pass through the second multiband mirror 120 and exit the chamber 104 as output beams 608. The output beams 608 may comprise the transmitted portions of the input beams 602 after they have completed the multi-pass reflection process within the chamber 104. Each input beam may be outputted through a respective outlet wedged window of the plurality of outlet wedged windows and through a respective outlet through-hole of the outlet through-holes 122. The output beams 608 may carry spectroscopic information about the gas sample that was present in the chamber 104 during the multi-pass reflection process.

The multiband multi-pass absorption spectroscopy gas sensing system 600 may include one or more fiber optic combiner couplers 610 configured to combine the output beams 608 from the multiband multi-pass absorption spectroscopy gas cell 100. For example, the multiband multi-pass absorption spectroscopy gas sensing system 600 may include one or two fiber optic combiner coupler(s) 610 configured to combine the output beams 608 from the multiband multi-pass absorption spectroscopy gas cell 100. The fiber optic combiner coupler(s) 610 may receive the multiple wavelength bands that exit the gas cell as separate output beams 608 and combine them into one or more optical outputs. For example, the fiber optic combiner coupler(s) 610 may receive the multiple wavelength bands that exit the gas cell as separate output beams 608 and combine them into one or more optical outputs 610 may maintain the spectroscopic information from each wavelength band while enabling efficient coupling to detection components.

As shown in FIG. 6, the system may include a broadband photoelectric detector 612 configured to receive combined output beams from the fiber optic combiner coupler(s) 610. The broadband photoelectric detector 612 may convert the optical signals from the fiber optic combiner coupler(s) 610 into electrical signals that may be processed for spectroscopic analysis. The broadband photoelectric detector 612 may have sensitivity across the multiple wavelength bands used in the system, enabling detection of spectroscopic information from ultraviolet, visible, near infrared, and short infrared wavelengths.

The multiband multi-pass absorption spectroscopy gas sensing system 600 may include a dual-comb spectroscopy processor 614 that may be configured to analyze signals from the broadband photoelectric detector 612. The dual-comb spectroscopy processor 614 may receive the electrical signals from the broadband photoelectric detector 612 and process them to restore the absorption spectroscopy features of target gases. The dual-comb spectroscopy processor 614 may analyze the spectroscopic data to determine the presence and concentration of target gases in the sample that was analyzed within the chamber 104.

In this regard, the method of multiband multi-pass absorption spectroscopy gas sensing may include dividing broadband light into a plurality of wavelength bands using the fiber optic wavelength division multiplexer 606. The method may include introducing the plurality of wavelength bands into the multiband multi-pass absorption spectroscopy gas cell 100 through the inlet wedged windows 113 and through the inlet through-holes 112 in the first multiband mirror 110, where the first multiband mirror 110 may have the plurality of concentric dielectric coated zones. The method may include reflecting each wavelength band multiple times between the first multiband mirror 110 and the second multiband mirror 120 within the chamber 104, where the second multiband mirror 120 may have the plurality of concentric dielectric coated zones.

The method may include outputting the plurality of wavelength bands from the multiband multi-pass absorption spectroscopy gas cell 100 through the outlet wedged windows 123 and through the outlet through-holes 122 in the second multiband mirror 120. The method may include combining the plurality of wavelength bands using the fiber optic combiner coupler(s) 610 and detecting the combined wavelength bands using the broadband photoelectric detector 612. The method may include processing detected signals to determine absorption spectroscopy features of target gases using the dual-comb spectroscopy processor 614, enabling comprehensive gas analysis across multiple spectral bands using a single integrated system.

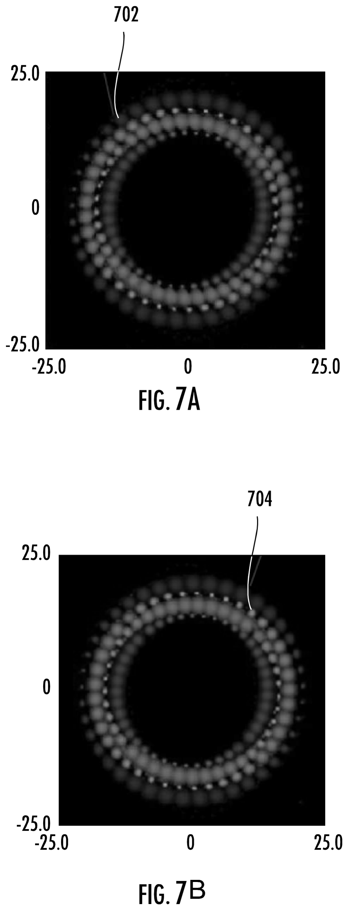

Referring to FIG. 7A and FIG. 7B, the multiband multi-pass absorption spectroscopy gas cell 100 may generate distinct reflection patterns on the mirror surfaces that demonstrate the controlled propagation of light beams during the multi-pass absorption process. The reflection patterns may illustrate how the input beams 602 interact with the first multiband mirror 110 and the second multiband mirror 120 to achieve multiple reflections while maintaining spectral separation and avoiding optical interference between different wavelength bands.

As shown in FIG. 7A, a concentric pattern 702 may be formed on the first multiband mirror 110, representing the input beam reflection pattern that occurs during the multi-pass absorption spectroscopy process. The concentric pattern 702 may demonstrate the spatial distribution of light reflections across the surface of the first multiband mirror 110, with the pattern comprising multiple rings arranged concentrically around a central point. Each ring within the concentric pattern 702 may correspond to successive reflections of the input beams 602 as they traverse the chamber 104 and interact with the concentric dielectric coated zones.

The concentric pattern 702 may illustrate how the input beams 602 create distinct reflection points on the first multiband mirror 110 during each reflection cycle. The rings within the concentric pattern 702 may be arranged such that the light paths do not overlap, thereby avoiding interference between different reflections and maintaining the integrity of the spectroscopic measurements. The spatial arrangement of the rings may be determined by the concave geometry of the first multiband mirror 110 and the positioning of the inlet through-holes 112 relative to the concentric dielectric coated zones.

With continued reference to FIG. 7A, the concentric pattern 702 may demonstrate how each wavelength band of the input beams 602 interacts with its corresponding coating zone on the first multiband mirror 110. The pattern may show that different wavelength bands create reflection points at different radial distances from the center of the first multiband mirror 110, corresponding to the first concentric dielectric coated zone 119a, second dielectric concentric coated zone 119b, third concentric dielectric coated zone 119c, and fourth concentric dielectric coated zone 119d. The radial separation of the reflection points may enable simultaneous operation of multiple wavelength bands without cross-interference.

As illustrated in FIG. 7B, a concentric pattern 704 may be formed on the second multiband mirror 120, representing the input beam reflection pattern that occurs as the light beams complete their multi-pass journey through the chamber 104. The concentric pattern 704 may demonstrate the spatial distribution of light reflections across the surface of the second multiband mirror 120, with the pattern comprising multiple rings arranged concentrically in a configuration that corresponds to the concentric pattern 702 on the first multiband mirror 110.

The concentric pattern 704 may illustrate how the input beams 602 create distinct reflection points on the second multiband mirror 120 during each reflection cycle before exiting through the outlet through-holes 122. Each ring within the concentric pattern 704 may correspond to successive reflections that have occurred during the multi-pass process, with the final reflections leading to the transmission of the output beams 608 (e.g., input beams 602 after completion of the multiple reflections within the chamber 104) through the outlet windows. In various embodiments, the output beams 608 refers to the input beams 602 after completion of the multiple reflections within the chamber 104. The concentric pattern 704 may demonstrate that the reflection geometry is maintained throughout the multi-pass process, ensuring consistent optical performance.

The concentric pattern 704 may show that the light beams maintain their spectral separation and spatial organization as they approach the exit points of the chamber 104. The rings within the concentric pattern 704 may be positioned at radial distances that correspond to the concentric dielectric coated zones on the second multiband mirror 120, enabling each wavelength band to exit through its appropriate outlet through-hole 122. The pattern may demonstrate that the multi-pass reflection process preserves the wavelength-specific characteristics of each beam while achieving the desired optical path length enhancement.

The concentric patterns 702 and 704 may collectively demonstrate that the multiband multi-pass absorption spectroscopy gas cell 100 achieves multiple reflections while maintaining distinct optical paths for each wavelength band. The spatial organization of the patterns show that the concave geometry of the first multiband mirror 110 and the second multiband mirror 120 enables controlled reflection sequences that maximize the optical path length without creating interference between different spectral channels. The concentric patterns 702 and 704 illustrate the effectiveness of the concentric dielectric coated zone configuration in enabling multiband operations. The patterns show that each coating zone maintains its spectral selectivity throughout the multi-pass process, with the reflection points remaining within their designated radial regions on both the first multiband mirror 110 and the second multiband mirror 120. The consistent spatial organization of the patterns may demonstrate that the multiband multi-pass absorption spectroscopy gas cell 100 provides stable and predictable optical performance across all wavelength bands.

The reflection patterns may provide visual confirmation that the wedge surface 117 configuration of the inlet windows and outlet windows effectively minimizes back reflection interference. The defined rings within the concentric patterns 702 and 704 may indicate that unwanted reflections are successfully suppressed, allowing the primary reflection sequences to proceed without degradation. The patterns may demonstrate that the optical design achieves the intended multi-pass performance while maintaining the spectral purity needed for accurate gas detection and analysis.

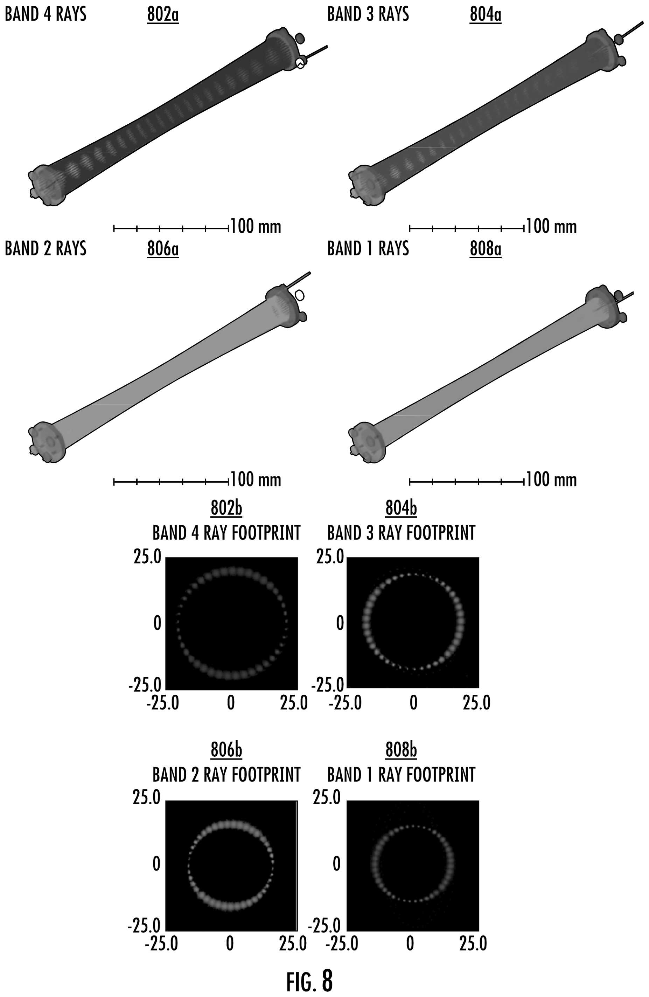

Referring to FIG. 8, the multiband multi-pass absorption spectroscopy gas cell 100 may generate band-specific ray patterns. The band-specific ray patterns may be defined by the spatial distribution of the spectral bands within the chamber 104. The reflection sequence of the band rays may be controlled by the concave geometry of the first multiband mirror 110 and the second multiband mirror 120 to achieve the desired optical path length enhancement. As shown in FIG. 8, Each band ray visualization may include a scale bar indicating the physical dimensions of the chamber 104, providing reference for the spatial extent of the reflection patterns.

Band 4 rays 802a depicted in FIG. 8 may correspond to a fourth spectral band within the multiband multi-pass absorption spectroscopy gas cell 100, showing how the fourth spectral band may propagate between the first multiband mirror 110 and the second multiband mirror 120 during a multi-pass reflection process. The band 4 rays 802a may interact with the fourth concentric dielectric coated zone 119d on both the first multiband mirror 110 and the second multiband mirror 120 to achieve high reflectance characteristics for the corresponding wavelength range. The reflection points along the band 4 rays 802a may be positioned at a radial location on the mirror surfaces, corresponding to the fourth concentric dielectric coated zone 119d that may be optimized for short infrared wavelengths. The spatial positioning of the band 4 rays 802a may enable the fourth spectral band to undergo multiple reflections without interfering with the optical paths of other wavelength bands. The controlled reflection sequence of the band 4 rays 802a may be optimized to provide enhanced optical path length for the short infrared wavelength range while avoiding interference with other spectral bands.

Band 4 ray footprint 802b depicted in FIG. 8 illustrates the reflection pattern formed by the reflections of the band 4 rays 802a on the mirror surfaces. The band 4 ray footprint 802b may define a circular pattern with reflection points arranged in a ring configuration while maintaining separation from the patterns created by other wavelength bands. For example, the band 4 ray footprint 802b may define a circular reflection pattern on the surfaces of the mirror that does not overlap with the patterns created by other wavelength bands. The band 4 ray footprint 802b may define a circular reflection pattern at a radial location on the surfaces of the mirrors corresponding to the fourth concentric dielectric coated zone 119d. The reflection points within the band 4 ray footprint 802b may correspond to the successive reflections that occur as the band 4 rays 802a traverse the chamber 104 multiple times between the mirrors.

Band 3 rays 804a depicted in FIG. 8 may represent the beam path for a third spectral band within the multiband multi-pass absorption spectroscopy gas cell 100, showing how the third spectral band may propagate between the first multiband mirror 110 and the second multiband mirror 120 during a multi-pass reflection process. The band 3 rays 804a may interact with the third concentric dielectric coated zone 119c on the mirror surfaces to achieve optimized reflectance for near infrared wavelengths. The band 3 rays 804a may be positioned at a different radial location compared to the band 4 rays 802a. For example, the reflection points along the band 3 rays 804a may be positioned at a radial location on the mirror surfaces, corresponding to the third concentric dielectric coated zone 119c that may be optimized for near infrared wavelengths. The spatial positioning of the band 3 rays 804a may enable the third spectral band to undergo multiple reflections without interfering with the optical paths of other wavelength bands. The controlled reflection sequence of the band 3 rays 804a may be optimized to provide enhanced optical path length for the near infrared wavelength range while avoiding interference with other spectral bands.

Band 3 ray footprint 804b depicted in FIG. 8 illustrates the reflection pattern formed by the band 3 rays 804a on the mirror surfaces. The band 3 ray footprint 804b may define a circular reflection pattern with reflection points arranged in a ring configuration. The band 3 ray footprint 804b may define a reflection pattern created by the third spectral band that maintains spatial separation from other wavelength bands while achieving multiple reflections within the chamber 104. The band 3 ray footprint 804b may define a circular reflection pattern at a radial location on the surfaces of the mirrors that correspond to the third concentric dielectric coated zone 119c. The reflection points within the band 3 ray footprint 804b may correspond to the successive reflections that occur as the band 3 rays 804a traverse the chamber 104 multiple times between the mirrors.

Band 2 rays 806a depicted in FIG. 8 may represent the beam path for a second spectral band within the multiband multi-pass absorption spectroscopy gas cell 100, showing how the second spectral band propagates between the first multiband mirror 110 and the second multiband mirror 120 during the multi-pass reflection process. The band 2 rays 806a may interact with the second dielectric concentric coated zone 119b on the mirror surfaces to achieve high reflectance for visible wavelengths. The band 2 rays 806a may be positioned at a radial location that corresponds to the second dielectric concentric coated zone 119b, which surrounds the first concentric dielectric coated zone 119a and is surrounded by the third concentric dielectric coated zone 119c. For example, the reflection points along the band 2 rays 806a may be positioned at a radial location on the mirror surfaces corresponding to the second dielectric concentric coated zone 119b that may be optimized for visible wavelengths. The spatial positioning of the band 2 rays 806a may enable the second spectral band to undergo multiple reflections without interfering with the optical paths of other wavelength bands. For example, the positioning of the band 2 rays 806a may enable the second spectral band to achieve multiple reflections while maintaining spatial separation from the other band rays. The controlled reflection sequence of the band 2 rays 806a may be optimized to provide enhanced optical path length for the visible wavelength range while avoiding interference with other spectral bands.

Band 2 ray footprint 806b depicted in FIG. 8 illustrates a reflection pattern formed by the band 2 rays 806a on the mirror surfaces. The band 2 ray footprint 806b may define a circular pattern with reflection points arranged in a ring configuration while maintaining separation from the patterns created by other wavelength bands. The band 2 ray footprint 806b may define a circular reflection pattern at a radial location on the surfaces of the mirrors corresponding to the second dielectric concentric coated zone 119b. The reflection points within the band 2 ray footprint 806b may correspond to the successive reflections that occur as the band 2 rays 806a traverse the chamber 104 multiple times between the mirrors.

Band 1 rays 808a depicted in FIG. 8 may represent the beam path for a first spectral band within the multiband multi-pass absorption spectroscopy gas cell 100, showing how the first spectral band propagates between the first multiband mirror 110 and the second multiband mirror 120 during the multi-pass reflection process. The band 1 rays 808a may interact with the first concentric dielectric coated zone 119a at the mirror surfaces to achieve optimized reflectance for ultraviolet wavelengths. The band 1 rays 808a may be positioned at the a radial location corresponding to the first concentric dielectric coated zone 119a. For example, the reflection points along the band 1 rays 808a may be positioned at a radial location on the mirror surfaces, corresponding to the second concentric dielectric coated zone 119a that may be optimized for ultraviolet wavelengths. The spatial positioning of the band 1 rays 808a may enable the first spectral band to undergo multiple reflections without interfering with the optical paths of other wavelength bands. For example, the positioning of the band 1 rays 808a may enable the first spectral band to achieve multiple reflections while maintaining spatial separation from the other band rays. The controlled reflection sequence of the band 1 rays 808a may be optimized to provide enhanced optical path length for the ultraviolet wavelength range while avoiding interference with other spectral bands.

Band 1 ray footprint 808b depicted in FIG. 8 illustrates the reflection pattern formed by the band 1 rays 808a on the mirror surfaces. The band 1 ray footprint 808b may define a circular reflection pattern with reflection points in a ring configuration while maintaining separation from the patterns created by other wavelength bands. The band 1 ray footprint 808b may define a reflection pattern at a radial location on the surfaces of the mirrors corresponding to the second dielectric concentric coated zone 119b. The reflection points within the band 1 ray footprint 808b may correspond to the successive reflections that occur as the band 1 rays 808a traverse the chamber 104 multiple times between the mirrors.

The band-specific ray patterns illustrated in FIG. 8 may collectively show that each wavelength band creates a distinct/different circular pattern on the mirror surfaces without overlap between different spectral bands. The spatial separation of the band 4 ray footprint 802b, band 3 ray footprint 804b, band 2 ray footprint 806b, and band 1 ray footprint 808b may enable multiple wavelength bands to be processed simultaneously within the multiband multi-pass absorption spectroscopy gas cell 100. The distinct patterns show that the concentric dielectric coated zone configuration successfully maintains spectral separation while enabling multiband operations.

The ray footprint patterns show that each spectral band may undergo a specific amount of reflections between the first multiband mirror 110 and the second multiband mirror 120, that enhances absorption sensitivity. For example, in some embodiments, the method of multiband multi-pass absorption spectroscopy gas sensing may involve reflecting each wavelength band multiple times, where reflecting each wavelength band multiple times may comprise reflecting each wavelength band approximately eighty times between the first multiband mirror 110 and the second multiband mirror 120. The eighty reflections may provide substantial enhancement of the optical path length, enabling improved detection sensitivity for gases with low concentrations or weak absorption features across all spectral bands.

The visualization of the band-specific ray patterns show that the multiband multi-pass absorption spectroscopy gas cell 100 maintains consistent optical performance across all wavelength bands while achieving the desired enhancement factor through multiple reflections. The distinct spatial organization of the reflection pattern of each band ray shows that the concentric dielectric coated zone configuration enables effective multiband operations without compromising the performance of individual spectral channels.

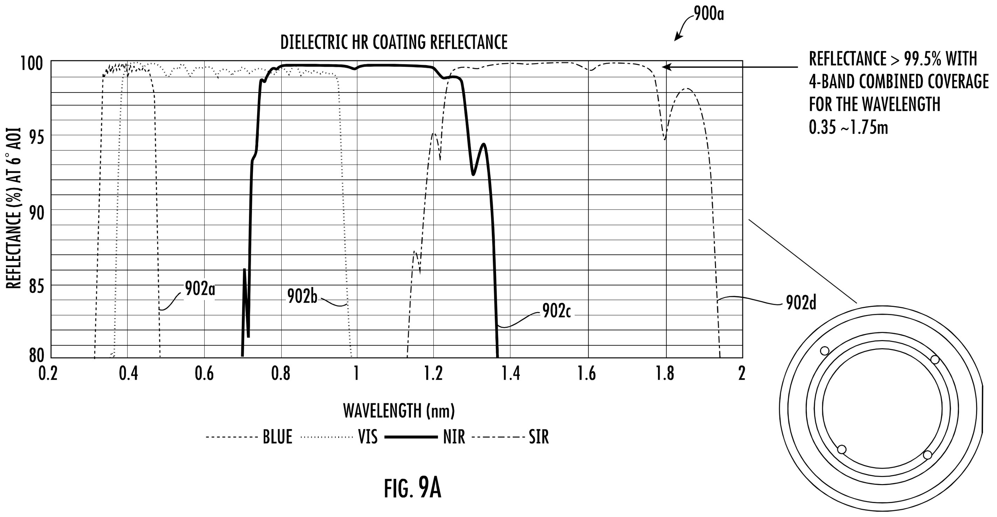

Referring to FIG. 9A, a dielectric coated mirror reflectance plot 900a is provided. The dielectric coated mirror reflectance plot 900a illustrates example reflectance performance characteristics of the concentric dielectric coated zones used in the first multiband mirror 110 and the second multiband mirror 120. The dielectric coated mirror reflectance plot 900a shows reflectance percentage as a function of wavelength across the electromagnetic spectrum, demonstrating how the multiband coating configuration achieves high reflectance values across multiple spectral bands simultaneously. The dielectric coated mirror reflectance plot 900a may provide quantitative data that supports the enhanced performance capabilities of the multiband multi-pass absorption spectroscopy gas cell 100.

As shown in FIG. 9A, the dielectric coated mirror reflectance plot 900a illustrates four distinct spectral bands where reflectance exceeds 99.5%, enabling effective multi-pass absorption spectroscopy across a broad wavelength range. The four spectral bands may correspond to the first concentric dielectric coated zone 119a, second dielectric concentric coated zone 119b, third concentric dielectric coated zone 119c, and fourth concentric dielectric coated zone 119d that are arranged concentrically on the mirror surfaces. Each spectral band may achieve reflectance values that significantly exceed the performance of conventional mirror coatings, enabling enhanced optical path length multiplication through increased numbers of reflections.

The dielectric coated mirror reflectance plot 900a depicts a first wavelength band 902a (e.g., near ultraviolet and blue wavelength band) encompassing near ultraviolet and blue wavelengths from approximately 0.35 to 0.45 microns. The first wavelength band 902a may correspond to the first concentric dielectric coated zone 119a positioned at the innermost zone in concentric coating arrangement, providing optimized reflectance characteristics for ultraviolet and blue wavelengths. The first wavelength band 902a may achieve reflectance values exceeding 99.5% across the near ultraviolet and blue wavelength range, enabling effective multi-pass absorption spectroscopy for gas species that exhibit absorption features in the ultraviolet and blue wavelength regions.

With continued reference to FIG. 9A, the dielectric coated mirror reflectance plot 900a depicts a second wavelength band 902b (e.g., visible wavelength band) encompassing wavelengths from approximately 0.4 to 0.9 microns. The second wavelength band 902b band may correspond to the second dielectric concentric coated zone 119b that surrounds the first concentric dielectric coated zone 119a on the mirror surfaces. The second wavelength band 902b may provide reflectance values exceeding 99.5% across the visible wavelength range, enabling enhanced sensitivity for gas detection applications that utilize visible light absorption spectroscopy. The visible wavelength band coverage may enable detection of gas species with absorption features in the visible spectrum while maintaining the high reflectance needed for effective multi-pass operations.

The dielectric coated mirror reflectance plot 900a depicts a third wavelength band 902c (e.g., a near-infrared wavelength band) encompassing wavelengths from approximately 0.8 to 1.25 micrometers. The third wavelength band 902c may correspond to the third concentric dielectric coated zone 119c that surrounds the second dielectric concentric coated zone 119b in the concentric arrangement. The third wavelength band 902c may achieve reflectance values exceeding 99.5% across the near infrared wavelength range, providing enhanced performance for gas sensing applications that target absorption features in the near-infrared spectral region. The near-infrared band may enable detection of various gas species that exhibit characteristic absorption lines in this wavelength range.

As illustrated in FIG. 9A, the dielectric coated mirror reflectance plot 900a depicts a fourth wavelength band 902d (e.g., short-infrared wavelength band) encompassing wavelengths from approximately 1.25 to 1.75 micrometers. The fourth wavelength band 902d may correspond to the fourth concentric dielectric coated zone 119d positioned at the outermost zone in the concentric coating arrangement. The short-infrared band may provide reflectance values exceeding 99.5% across the 1.25 to 1.75 micrometer range, enabling effective multi-pass absorption spectroscopy for gas species with absorption features in the short-infrared wavelength region. The fourth wavelength band 902d may complete the multiband coverage by addressing longer wavelength applications that may be relevant for comprehensive gas detection capabilities.

The dielectric coated mirror reflectance plot 900a shows that the combined coverage of the four spectral bands achieves reflectance greater than 99.5% for wavelengths ranging from 0.35 to 1.75 micrometers. The combined coverage may provide continuous high-reflectance performance across a broad spectral range that encompasses ultraviolet, visible, near-infrared, and short-infrared wavelengths. The 4-band combined coverage may enable the multiband multi-pass absorption spectroscopy gas cell 100 to accommodate diverse gas sensing applications that require different wavelength ranges for optimal detection sensitivity.

In some examples, the high reflectance values shown in the dielectric coated mirror reflectance plot 900a may enable the multiband multi-pass absorption spectroscopy gas cell 100 to achieve approximately eighty reflections for each wavelength band without significant optical losses. The reflectance values exceeding 99.5% may provide substantial improvement compared to conventional mirrors. The enhanced reflectance characteristics may enable higher enhancement factors and improved detection sensitivity across all spectral bands.

The wavelength coverage demonstrated in the dielectric coated mirror reflectance plot 900a may enable the multiband multi-pass absorption spectroscopy gas cell 100 to detect a wide variety of gas species that exhibit absorption features across the ultraviolet to short-infrared spectral range. The broad wavelength coverage may provide comprehensive gas sensing capabilities that may not be achievable with conventional single-coating mirror systems. The high reflectance values across all four spectral bands may ensure that each wavelength range receives adequate enhancement through the multi-pass reflection process, enabling consistent detection performance across the entire spectral range.

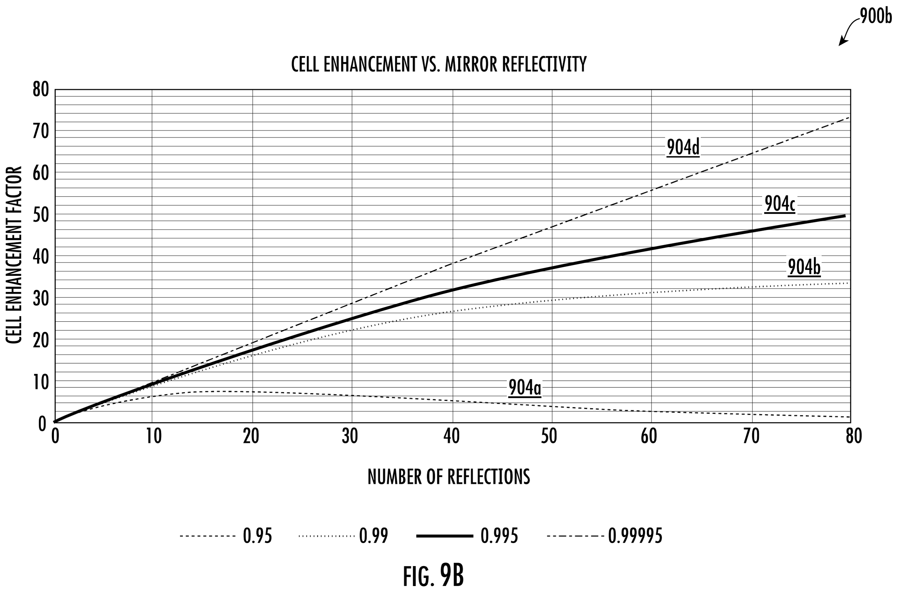

FIG. 9B illustrates an example mirror reflectivity plot 900b. Specifically, FIG. 9B illustrates the relationship between number of mirror reflections and the cell enhancement factor for different mirror types/configurations and/or reflectance values. The mirror reflectivity plot 900b illustrates how the reflectance characteristics of the mirrors of the gas cell 100 may impact the performance capabilities of the multiband multi-pass absorption spectroscopy gas cell 100. The mirror reflectivity plot 900b may provide quantitative analysis that supports the selection of high-reflectance dielectric coatings for achieving enhanced gas detection sensitivity through extended optical path lengths.

As shown in FIG. 9B, the x-axis of the mirror reflectivity plot 900b represents the number of mirror reflections and the y-axis represents the cell enhancement factor. The mirror reflectivity plot 900b depicts four distinct reflectance curves 904a-d that represent different mirror reflectivity values, illustrating the impact that reflectance performance has on the overall enhancement capabilities of the gas cell system.

The mirror reflectivity plot 900b includes a first reflectance curve 904a representing a mirror reflectance of 0.95, which may correspond to the performance characteristics of conventional gold-coated mirrors. The first reflectance curve 904a may provide limited enhancement capabilities, particularly as the number of mirror reflections increases beyond approximately twenty reflections. The first reflectance curve 904a shows that conventional gold-coated mirrors with 0.95 reflectivity may achieve only minimal enhancement factors even with moderate numbers of reflections, illustrating the limitations of conventional mirror technologies for high-performance gas sensing applications.

With continued reference to FIG. 9B, the mirror reflectivity plot 900b includes a second reflectance curve 904b representing a mirror reflectance of 0.99, which may demonstrate improved enhancement performance compared to the first reflectance curve 904a. The second reflectance curve 904b may illustrate how modest improvements in mirror reflectance may result in improved enhancement factors, particularly as the number of reflections increases. The plot 900b shows that 0.99 reflectance enables more effective multi-pass operations compared to conventional mirror coatings, though the enhancement factor may still be limited compared to higher reflectivity values.

The mirror reflectivity plot 900b include a third reflectance curve 904c representing a mirror reflectance of 0.995, which may correspond to the performance characteristics achievable with the dielectric mirror such as the dielectric coated mirrors described above. The third reflectance curve 904c shows enhanced performance compared to lower reflectivity values, illustrating how the high-reflectance dielectric coatings, as described above, may enable effective multi-pass operations with substantial enhancement factors. The third reflectance curve 904c illustrate that 0.995 reflectance provides a substantial improvement in enhancement capabilities that enables practical implementation of high-reflection-count gas sensing systems. Specifically, the third reflectance curve 904c shows the a dielectric mirror or dielectric coated mirror may achieve 50 times gas sensing enhancement (e.g., enhancement factor of 50) with 80 mirror reflections.

As illustrated in FIG. 9B, the mirror reflectivity plot 900b includes a fourth reflectance curve 904d representing a mirror reflectivity of 0.99995, which may represent near-ideal mirror performance that approaches theoretical limits for reflection-based enhancement systems. The fourth reflectance curve 904d may show that extremely high reflectivity values enable substantial enhancement factors even with large numbers of reflections, approaching the theoretical limit where enhancement factor equals the number of reflections.

As described above, the dielectric coated mirrors may achieve an enhancement factor of 50 with 80 mirror reflections. The enhancement factor of approximately 50 may represent a substantial improvement compared to conventional mirror systems while remaining achievable with practical dielectric coating technologies. The 0.995 reflectance may enable the multiband multi-pass absorption spectroscopy gas cell 100 to achieve enhanced gas detection sensitivity across all four spectral bands.

At eighty reflections, the first reflectance curve (e.g., 0.95 reflectance curve) may achieve a gas sensing enhancement factor of approximately 2, demonstrating the limitations of conventional gold-coated mirrors for high-reflection-count applications. At eighty reflections, the second reflectance curve (e.g., 0.99 mirror reflectance) may achieve a cell enhancement factor of approximately 33, demonstrating improved performance compared to conventional mirrors but reduced enhancement compared to the higher reflectance mirrors such as dielectric coated mirrors described herein.

The mirror reflectivity plot 900b shows that gas sensing enhancement factor becomes increasingly sensitive to mirror reflectivity as the number of reflections increases. The reflectance curves show that small differences in reflectance values may result in substantial differences in gas sensing enhancement when operating with high reflection counts such as in various multi-pass absorption spectroscopy applications. The plot 900b shows that the 0.995 reflectance achieved by the dielectric coated mirrors provides a practical balance between achievable coating performance and meaningful enhancement capabilities.

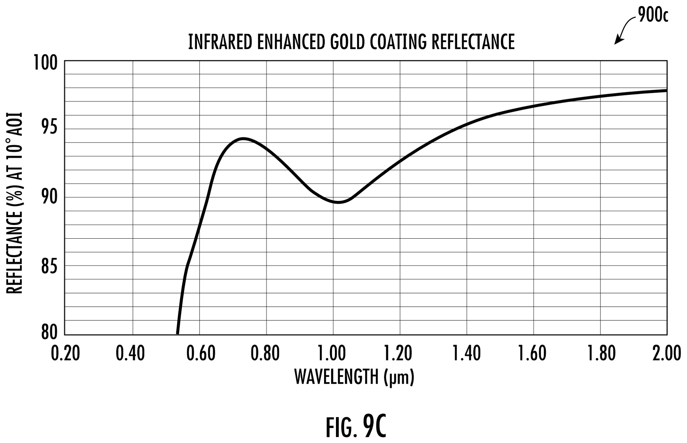

As shown in gold coating reflectance plot 900c depicted in FIG. 9C, gold coated mirrors can only provide 95% reflectance in IR wavelength (>1,4um) for limited multi-pass absorption spectroscopy gas sensing application. FIG. 9C further shows that gold coated mirrors can only provide 90% reflectance in deep red and near IR wavelength (>0.65um), too low for multi-pass application and cannot provide usable reflectance in most of the visible wavelength (<0.65um)

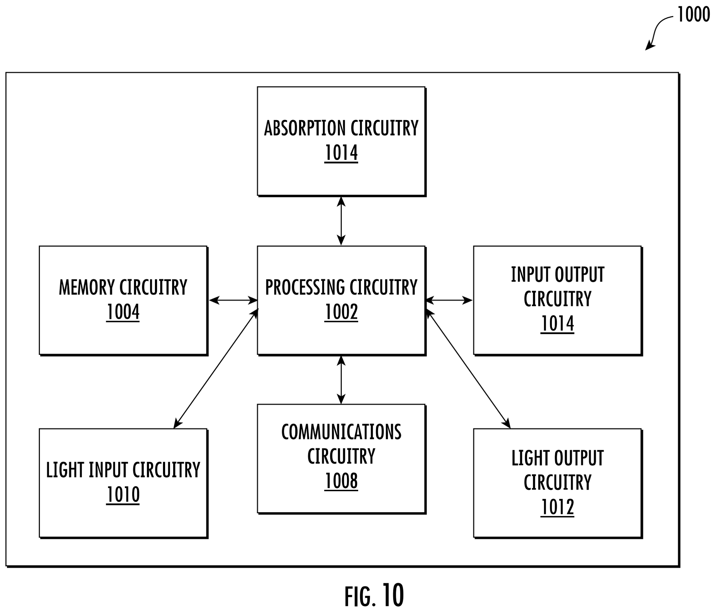

FIG. 10 illustrates a block diagram of an example apparatus that may be specially configured in accordance with an example embodiment of the present disclosure. Specifically, FIG. 2 depicts an example computing apparatus 1000 ("apparatus 1000") specially configured in accordance with at least some example embodiments of the present disclosure. In some embodiments, the system 600, and/or a portion thereof is embodied by one or more system(s), such as the apparatus 1000 as depicted and described in FIG. 10.

The apparatus 1000 may comprise a processor or processing circuitry 1002, memory circuitry 1004, input/output circuitry 1006, and communications circuitry 1008, light input circuitry 1010, and/or light output circuitry 1012. In some embodiments, one or more portions of the apparatus 1000 (e.g., one or more of the components thereof) are configured to execute and perform the operations described herein.

Although components are described with respect to functional limitations, it should be understood that at least some of the particular implementations necessarily include the use of particular computing hardware. It should also be understood that in some embodiments certain of the components described herein include similar or common hardware. For example, in some embodiments two sets of circuitries both leverage use of the same processor(s), memory(ies), circuitry(ies), and/or the like to perform their associated functions such that duplicate hardware is not required for each set of circuitries.

Processing circuitry 1002 may be embodied in a number of different ways. In various embodiments, the use of the terms processor or processing circuitry should be understood to include a single core processor, a multi-core processor, multiple processors internal to the example apparatus 1000, and/or one or more remote or cloud processor(s) external to the example apparatus 1000. In some example embodiments, processing circuitry 1002 may include one or more processing devices configured to perform independently. Alternatively, or additionally, processing circuitry 1002 may include one or more processor(s) configured in tandem via a bus to enable independent execution of operations, instructions, pipelining, and/or multithreading.

In an example embodiment, the processing circuitry 1002 may be configured to execute instructions stored in the memory circuitry 1004 or otherwise accessible to the processor. Alternatively, or additionally, the processing circuitry 1002 may be configured to execute hard-coded functionality. As such, whether configured by hardware or software methods, or by a combination thereof, processing circuitry 1002 may represent an entity (e.g., physically embodied in circuitry) capable of performing operations according to embodiments of the present disclosure while configured accordingly. Alternatively, or additionally, processing circuitry 1002 may be embodied as an executor of software instructions, and the instructions may specifically configure the processing circuitry 1002 to perform the various algorithms embodied in one or more operations described herein when such instructions are executed. In some embodiments, the processing circuitry 1002 includes hardware, software, firmware, and/or a combination thereof that performs one or more operations described herein.

The processing circuitry 1002 may implement various signal processing algorithms to extract meaningful absorption data from the received optical signals. The processing circuitry 1002 may perform baseline correction to account for variations in light source intensity and detector response characteristics. The processing circuitry 1002 may apply digital filtering techniques to reduce noise and improve signal-to-noise ratios in the absorption measurements. The processing circuitry 1002 may execute spectral analysis algorithms that identify characteristic absorption peaks corresponding to specific molecular transitions of target gases.

In some embodiments, the processing circuitry 1002 (and/or co-processor or any other processing circuitry assisting or otherwise associated with the processor) is/are in communication with the memory circuitry 1004 via a bus for passing information among components of the example apparatus 1000.

Memory or memory circuitry 1004 may be non-transitory and may include, for example, one or more volatile and/or non-volatile memories. In some embodiments, the memory circuitry 1004 includes or embodies an electronic storage device (e.g., a computer readable storage medium). In some embodiments, the memory circuitry 1004 is configured to store information, data, content, applications, instructions, or the like, for enabling the example apparatus 1000 to carry out various operations and/or functions in accordance with example embodiments of the present disclosure.

The memory circuitry 1004 may store reference absorption spectra for various target gases, enabling the processing circuitry 1002 to perform pattern matching and identification of unknown gas components. The memory circuitry 1004 may maintain calibration data that relates measured absorption signals to actual gas concentrations, accounting for factors such as temperature, pressure, and optical path length variations. The memory circuitry 1004 may store historical measurement data for trend analysis and long-term monitoring applications. The memory circuitry 1004 may include both volatile memory for real-time data processing and non-volatile memory for persistent storage of calibration parameters and reference data.

Input/output circuitry 1006 may be included in the example apparatus 1000. In some embodiments, input/output circuitry 1006 may provide output to the user and/or receive input from a user. The input/output circuitry 1006 may be in communication with the processing circuitry 1002 to provide such functionality. The input/output circuitry 1006 may comprise one or more user interface(s). In some embodiments, a user interface may include a display that comprises the interface(s) rendered as a web user interface, an application user interface, a user device, a backend system, or the like. In some embodiments, the input/output circuitry 1006 also includes a keyboard, a mouse, a joystick, a touch screen, touch areas, soft keys a microphone, a speaker, or other input/output mechanisms. The processing circuitry 1002 and/or input/output circuitry 1006 may be configured to control one or more operations and/or functions of one or more user interface elements through computer program instructions (e.g., software and/or firmware) stored on a memory accessible to the processor (e.g., memory circuitry 1004, and/or the like). In some embodiments, the input/output circuitry 1006 includes or utilizes a user-facing application to provide input/output functionality to a computing device and/or other display associated with a user. In some embodiments, the input/output circuitry 1006 one or more indicator lights or the like for providing a user notification (e.g., an alert or warning).

Communications circuitry 1008 may be included in the example apparatus 1000. The communications circuitry 1008 may include any means such as a device or circuitry embodied in either hardware or a combination of hardware and software that is configured to receive and/or transmit data from/to a network and/or any other device, circuitry, or module in communication with the example apparatus 1000. In some embodiments the communications circuitry 1008 includes, for example, a network interface for enabling communications with a wired or wireless communications network. Additionally, or alternatively, the communications circuitry 1008 may include one or more network interface card(s), antenna(s), bus(es), switch(es), router(s), modem(s), and supporting hardware, firmware, and/or software, or any other device suitable for enabling communications via one or more communications network(s). In some embodiments, the communications circuitry 1008 may include circuitry for interacting with an antenna(s) and/or other hardware or software to cause transmission of signals via the antenna(s) and/or to handle receipt of signals received via the antenna(s). In some embodiments, the communications circuitry 1008 enables transmission to and/or receipt of data from a user device, one or more sensors, and/or other external computing device(s) in communication with the example apparatus 1000.

In some embodiments, the apparatus 1000 includes a light input circuitry 1010. The light input circuitry 1010 may include hardware components, software components, and/or a combination thereof configured to, with the processor 1002, memory 1004, input/output circuitry 1006 and/or communications circuitry 1008, perform one or more functions associated with a broadband dual-comb laser source 604 and/or fiber optic wavelength division multiplexer 606 (as described above with reference to FIG. 6). In some embodiments, the light input circuitry 1010 comprises a broadband dual-comb laser source such as the broadband dual-comb laser source 604 and comprises a fiber optic wavelength division multiplexer such as the fiber optic wavelength division multiplexer 606

In some embodiments, the apparatus 1000 includes a light output circuitry 1012. The light input circuitry 1010 may include hardware components, software components, and/or a combination thereof configured to, with the processor 1002, memory 1004, input/output circuitry 1006 and/or communications circuitry 1008, perform one or more functions associated with a fiber optic combiner coupler(s) 610, broadband photoelectric detector 612, and/or the dual-comb spectroscopy processor 614 (as described above with reference to FIG. 6). In some embodiments, the light output circuitry 1012 comprises a fiber optic combiner coupler(s) such as the fiber optic combiner coupler 610, broadband photoelectric detector such as the broadband photoelectric detector 612, and/or a dual-comb spectroscopy processor such as the dual-comb spectroscopy processor 614

In some embodiments, the apparatus 1000 includes an absorption circuitry 1014. The absorption circuitry 1014 may include hardware components, software components, and/or a combination thereof configured to, with the processor 1002, memory 1004, input/output circuitry 1006 and/or communications circuitry 1008, perform one or more functions associated with a multiband multi-pass absorption spectroscopy gas cell 100 as described herein. In some embodiments, the absorption circuitry 1014 comprises a multiband multi-pass absorption spectroscopy gas cell 100.

In some embodiments, two or more of the sets of circuitries 1002-1014 are combinable. Alternatively, or additionally, one or more of the sets of circuitries 1002-1014 perform some or all of the operations and/or functionality described herein as being associated with another circuitry. In some embodiments, two or more of the sets of circuitries 1002-1014 are combined into a single module embodied in hardware, software, firmware, and/or a combination thereof.

While the description above provides an apparatus 1000, it is noted that the scope of the present disclosure is not limited to the description above. In some examples, an example apparatus 1000 in accordance with the present disclosure may be in other forms. In some examples, an example apparatus 1000 may comprise one or more additional and/or alternative elements, and/or may be structured differently than that illustrated in FIG. 10.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some cases one or more of the steps depicted may occur substantially simultaneously, or additional steps may be involved. Although specific terms are employed herein, they are used in a generic and descriptive sense only and not for purposes of limitation.

## Claims

1. A multiband multi-pass absorption gas cell comprising:
a housing defining a chamber for receiving a gas sample to be analyzed;
a first multiband mirror positioned at a first end of the housing, the first multiband mirror defining a plurality of inlet through-holes and comprising a first plurality of concentric dielectric coated zones corresponding to a plurality of wavelength bands;
a second multiband mirror positioned at a second end of the housing opposite the first end, the second multiband mirror comprising a plurality of outlet through-holes and a second plurality of concentric dielectric coated zones corresponding to the plurality of wavelength bands;
a plurality of inlet wedged windows positioned at the first end of the housing; and
a plurality of outlet wedged windows positioned at the second end of the housing.

2. The multiband multi-pass absorption gas cell of claim 1, further comprising:
an inlet for introducing the gas sample into the chamber; and
and an outlet for removing the gas sample from the chamber.

3. The multiband multi-pass absorption gas cell of claim 1, wherein each of the first multiband mirror and the second multiband mirror defines an inner surface and an outer surface, wherein the inner surface of the first multiband mirror and the inner surface of the second multiband mirror face each other, and wherein the inner surface of the first multiband mirror comprises the first plurality of concentric dielectric coated zones and the inner surface of the second multiband mirror comprises the second plurality of concentric dielectric coated zones.

4. The multiband multi-pass absorption gas cell of claim 3, wherein the plurality of inlet wedged windows are positioned adjacent the outer surface of the first multiband mirror and the plurality of outlet wedged windows are positioned adjacent to the outer surface of the second multiband mirror.

5. The multiband multi-pass absorption gas cell of claim 3, wherein each inlet through-hole of the plurality of inlet through-holes is conical and defines a first opening on the inner surface of the first multiband mirror and a second opening on the outer surface of the first multiband mirror that is larger than the first opening.

6. The multiband multi-pass absorption gas cell of claim 3, wherein each outlet through-holes of the plurality of outlet through-holes is conical and defines a first opening on the inner surface of the second multiband mirror and a second opening on the outer surface of the second multiband mirror that is larger than the first opening.

7. The multiband multi-pass absorption gas cell of claim 1, wherein the plurality of inlet through-holes of the first multiband mirror are defined within different dielectric coated zones of the first plurality of concentric dielectric coated zones, and wherein the plurality of outlet through-holes of the second multiband mirror are defined within different dielectric coated zones of the second plurality of concentric dielectric coated zones.

8. The multiband multi-pass absorption gas cell of claim 1, wherein the plurality of inlet through-holes of the first multiband mirror are defined at different radial distance from a center of the first multiband mirror, and wherein the plurality of outlet through-holes of the second multiband mirror are defined at different radial distance from a center of the second multiband mirror.

9. The multiband multi-pass absorption gas cell of claim 1, wherein the first plurality of concentric dielectric coated zones and the second plurality of concentric dielectric coated zones each comprise four concentric dielectric coated zones configured to provide greater than 99.5% reflectance in each of a plurality of wavelength bands.

10. The multiband multi-pass absorption gas cell of claim 8, wherein the plurality of wavelength bands comprise ultraviolet wavelength band, visible wavelength band, near infrared wavelength band, and short infrared wavelength band.

11. The multiband multi-pass absorption gas cell of claim 1, wherein the first multiband mirror and the second multiband mirror comprise a concave mirror.

12. A multiband multi-pass absorption gas sensing system comprising:
a broadband dual-comb laser source configured to emit light;
a fiber optic wavelength division multiplexer configured to generate a plurality of light beams from the light, wherein the plurality of light beams correspond to a plurality of wavelength bands; and
multiband multi-pass absorption gas cell for configured to receive the plurality of light beams, the multiband multi-pass absorption gas cell comprising:
a housing defining a chamber for receiving a gas sample to be analyzed;
a first multiband mirror positioned at a first end of the housing, the first multiband mirror defining a plurality of inlet through-holes and comprising a first plurality of concentric dielectric coated zones corresponding to a plurality of wavelength bands;
a second multiband mirror positioned at a second end of the housing opposite the first end, the second multiband mirror comprising a plurality of outlet through-holes and a second plurality of concentric dielectric coated zones corresponding to the plurality of wavelength bands;
a plurality of inlet wedged windows positioned at the first end of the housing; and
a plurality of outlet wedged windows positioned at the second end of the housing.

13. The multiband multi-pass absorption gas sensing system of claim 12, further comprising a fiber optic combine coupler configured to receive output light beams from the multiband multi-pass absorption gas cell and generate a single output beam.

14. The multiband multi-pass absorption gas sensing system of claim 13, further comprising a broadband photoelectric detector and a dual comb spectroscopy processor.

15. The multiband multi-pass absorption gas sensing system of claim 12, further comprising:
an inlet for introducing the gas sample into the chamber; and
and an outlet for removing the gas sample from the chamber.
